# EUROPEAN PATENT APPLICATION

(11) **EP 2 301 659 A1**
(43) Date of publication of application: **30.03.2011**
(21) Application number: 09758240.7
(22) Date of filing: 26.05.2009
(51) Int. Cl.: B01J 13/14, A23L 1/00, A61K 9/50, A61K 47/02, A61K 47/36, A61K 47/44, A23L 1/30

(54) **MICROCAPSULES, METHOD OF PRODUCING THE MICROCAPSULES AND FOOD AND DRINK CONTAINING THE MICROCAPSULES**

(30) Priority: 02.06.2008 JP 2008144885
(71) Applicant: Niigata University, Niigata-shi Niigata 950-2181 (JP); Sapporo Breweries Limited, Tokyo 150-8522 (JP)
(72) Inventor: TANAKA Masato, Niigata-shi Niigata 950-2181 (JP); TSUCHIMOTO Norihiko, Tokyo 150-8522 (JP)
(74) Representative: Vossius & Partner
(86) International application number: PCT/JP2009/059611
(87) International publication number: WO 2009/147973

(57) **Abstract**

A method of producing microcapsules that comprises an emulsification step in which a fat-soluble substance and a sodium alginate aqueous solution are mixed to obtain an emulsified liquid in which there are dispersed emulsified particles composed of the fat-soluble substance and having a mean particle size of not greater than 800 nm, and a spraying step in which the emulsified liquid is sprayed into a calcium ion-containing solution to obtain microcapsules in which the emulsified particles are encapsulated.

## Description

### Technical Field

The present invention relates to microcapsules, a method of producing the microcapsules, and a food and drink containing the microcapsules.

### Background Art

Microcapsules have a covering layer as a shell substance formed around a functional encapsulated substance serving as the core substance, to protect it, and they usually have particle sizes of about 100 µm-1 mm. In recent years, microcapsules have come to be used in a variety of fields, by combining encapsulated core substances and shell substances that cover the core substances. Production of microcapsules is being studied in particular for use in foods, medicines and the like (see Patent literatures 1-7).

### Citation List

### Patent Literature

[Patent literature 1] Japanese Unexamined Patent Application Publication HEI No. 05-049899
[Patent literature 2] Japanese Patent Public Inspection No. 2002-511796
[Patent literature 3] Japanese Unexamined Patent Application Publication No. 2000-185229
[Patent literature 4] Japanese Unexamined Patent Application Publication HEI No. 06-254382
[Patent literature 5] Japanese Unexamined Patent Application Publication HEI No. 05-049433
[Patent literature 6] Japanese Unexamined Patent Application Publication HEI No. 07-328416
[Patent literature 7] Japanese Unexamined Patent Application Publication No. 2007-290997

### Summary of Invention

### Technical Problem

Microcapsules used for foods or medicines are required to be microcapsules with small particle sizes and nearly spherical shapes, from the viewpoint of improving the feel of the microcapsules in the mouth/throat and stomach. With the methods described in Patent literatures 1-7, however, it is difficult to obtain microcapsules with particle sizes of less than 100 µm, and if the particle sizes of the microcapsules are reduced, concavities and convexities are produced in the resulting microcapsules and it can be difficult to obtain spheres.

Microcapsules for foods or medicines are also known which comprise fat-soluble substances enclosed in alginate gels. When such microcapsules are produced, with simple admixture of a fat-soluble substance and sodium alginate, it is difficult to obtain a microdispersed emulsified liquid of the fat-soluble substance and the particle sizes of the obtained microcapsules tend to be 100 µm or greater, while it is also difficult for the microcapsules to adopt spherical shapes.

It is an object of the present invention to provide spherical microcapsules with small particle sizes, as well as a method of producing the microcapsules and foods and drinks containing the microcapsules.

### Solution to Problem

The invention provides a method of producing microcapsules that comprises an emulsification step in which a fat-soluble substance and a sodium alginate aqueous solution are mixed to obtain an emulsified liquid in which there are dispersed emulsified particles composed of the fat-soluble substance and having a mean particle size of not greater than 800 nm, and a spraying step in which the emulsified liquid is sprayed into a calcium ion-containing solution to obtain microcapsules in which the emulsified particles are encapsulated.

The invention further provides a method of producing microcapsules wherein droplets of an emulsified liquid comprising emulsified particles composed of a fat-soluble substance and having a mean particle size of not greater than 800 nm dispersed in a sodium alginate aqueous solution, are contacted with a calcium ion-containing solution, to obtain microcapsules in which emulsified particles are encapsulated in a calcium alginate gel.

According to the invention, an emulsified liquid comprising microdispersed emulsified particles with a mean particle size of not greater than 800 nm is used, so that not only are the microcapsule particle sizes limited to less than 100 nm, but formation of concavities and convexities is inhibited as well. This will allow the microcapsules obtained according to the invention to have reduced particle sizes, and to be spherical. Such microcapsules also have high contained ratio for the fat-soluble substance, and excellent durability as well.

In the method of producing microcapsules described above, the emulsified particles are preferably dispersed under pressure in order to allow formation of fine emulsified particles. In order to allow formation of even finer emulsified particles, the emulsified particles are more preferably dispersed under a pressure of at least 5 MPa.

If the viscosity of the sodium alginate aqueous solution is 5 mPa·s or greater, the particle sizes of the obtained microcapsules can be made even smaller and spherical shapes can be obtained.

In the method of producing microcapsules according to the invention, the calcium ion-containing solution is preferably a calcium chloride aqueous solution, a calcium lactate aqueous solution or a calcium sulfate aqueous solution. This will allow instantaneous encapsulation of the emulsified particles, to result in even smaller particle sizes and to allow spherical microcapsules to be obtained.

The invention also provides microcapsules obtainable by the production method described above. The invention yet further provides microcapsules encapsulating a fat-soluble substance, wherein the microcapsules have the mean particle size of less than 100 µm and the degree of deformation of less than 1.20. Such microcapsules have small particle sizes and are spherical, while having high contained ratio for fat-soluble substances and excellent durability.

The invention further provides foods and drinks containing the microcapsules of the invention. Since such foods and drinks contain spherical microcapsules with small particle sizes, they have excellent feel of the microcapsules in the mouth/throat and stomach. Moreover, since the microcapsules also have excellent durability, leakage of the fat-soluble substance into foods and drinks can be inhibited, thus preventing reduction in the quality of the foods and drinks.

### Advantageous Effects of Invention

According to the invention, it is possible to provide spherical microcapsules with small particle sizes, as well as a method of producing the microcapsules and foods and drinks containing the microcapsules.

### Brief Description of Drawings

Fig. 1 is a schematic view showing a method of producing microcapsules according to the invention.
Fig. 2 is a schematic view showing microcapsules formed by the production method according to an embodiment of the invention.
Fig. 3 is a schematic view showing microcapsules formed by a conventional method.
Fig. 4 is a graph showing the particle size distribution of the microcapsules formed in Example 1.
Fig. 5 is a graph showing the particle size distribution of the microcapsules formed in Comparative Example 1.
Fig. 6 is an optical microscope photograph of the microcapsules formed in Example 1.
Fig. 7 is an optical microscope photograph of the microcapsules formed in Example 2.
Fig. 8 is an optical microscope photograph of the microcapsules formed in Comparative Example 1.
Fig. 9 is a photograph of the microcapsules formed in Example 13.
Fig. 10 is a photograph of the microcapsules formed in Example 15.
Fig. 11 is a photograph of the microcapsules formed in Example 16.
Fig. 12 is a photograph of the microcapsules formed in Example 18.
Fig. 13 is a graph showing the vitamin E residue ratio in microcapsules after a durability test.

### Description of Embodiments

Preferred embodiments of the invention will now be explained in detail, with reference to the accompanying drawings as necessary. Throughout the drawings, corresponding elements will be referred to by like reference numerals and will be explained only once. Unless otherwise specified, the vertical and horizontal positional relationships are based on the positional relationships in the drawings. Also, the dimensional proportions depicted in the drawings are not necessarily limitative.

### (Method of producing microcapsules)

The invention provides a method of producing microcapsules that comprises an emulsification step in which a fat-soluble substance and a sodium alginate aqueous solution are mixed to obtain an emulsified liquid in which there are dispersed emulsified particles composed of the fat-soluble substance and having a mean particle size of not greater than 800 nm, and a spraying step in which the emulsified liquid is sprayed into a calcium ion-containing solution to obtain microcapsules in which the emulsified particles are encapsulated. The invention further provides a method of producing microcapsules that comprises a step in which droplets of an emulsified liquid comprising emulsified particles composed of a fat-soluble substance and having a mean particle size of not greater than 800 nm dispersed in a sodium alginate aqueous solution, are contacted with a calcium ion-containing solution, to obtain microcapsules in which emulsified particles are encapsulated in a calcium alginate gel.

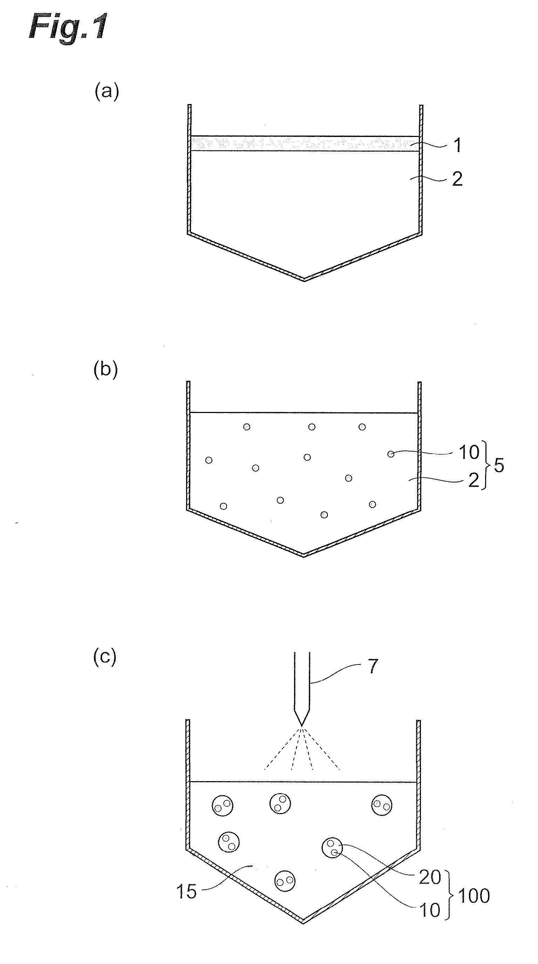

Fig. 1 is schematic view showing a method of producing microcapsules 100 according to an embodiment of the invention. The method of producing the microcapsules 100 of this embodiment will now be explained, with reference to Fig. 1.

### <Emulsification step>

First, an oil layer composed of a fat-soluble substance (hereunder referred to simply as "fat-soluble substance") 1 and a sodium alginate aqueous solution 2 are prepared, as shown in Fig. 1(a), and the fat-soluble substance 1 is dispersed in the sodium alginate aqueous solution 2 by mixing (hereunder also referred to as "pre- emulsification"). The method of pre-emulsification may be a method known in the prior art, and for example, a homomixer or homogenizer may be used for mixing and dispersion.

Next, the pre-emulsified dispersion is mixed by a high-speed, high-pressure method to obtain an emulsified liquid 5 in which the emulsified particles 10 composed of the fat-soluble substance 1 are microdispersed in the sodium alginate aqueous solution 2, as shown in Fig. 1(b) (hereunder also referred to as "emulsification").

The method of forming the emulsified liquid 5 is preferably one in which the fat-soluble substance 1 is microdispersed in the sodium alginate aqueous solution 2 while applying a high shear force. The apparatus used for emulsification is preferably, for example, a high-pressure homogenizer, nanomizer, homomixer, colloid mill, Disper mill or static mixer, and more preferably a high-pressure homogenizer or nanomizer. As the conditions for using a high-pressure homogenizer or nanomizer, the pressure is set to preferably 5 MPa or higher, more preferably 5-200 MPa and even more preferably 10-200 MPa, from the viewpoint of lowering the interfacial tension and further increasing the emulsifying power. From the same viewpoint, the temperature is set to preferably 10-80°C, more preferably 20-75°C and even more preferably 30-70°C.

The mean particle size of the emulsified particles 10 is not greater than 800 nm, more preferably not greater than 500 nm and even more preferably not greater than 300 nm. If the mean particle size of the emulsified particles 10 is greater than 800 nm, it will be difficult to reduce the microcapsule particle sizes, concavities and convexities will form in the microcapsules, and spherical shapes will not be easily obtained. The mean particle size of the emulsified particles 10 can be measured using a dynamic light scattering distribution meter, and it is referred to as the weight-average particle size.

The fat-soluble substance 1 may be a fat-soluble bioactive substance, and examples include coenzyme Q compounds such as ubiquinone, fat-soluble vitamins such as vitamin A, vitamin D, vitamin E and vitamin K compounds, and astaxanthin, zeaxanthin, fucoxanthin, β-carotene, DHA, EPA, and the like. Vitamin A compounds include retinol, retinoic acid, retinoids and carotenes, vitamin D compounds include cholecalciferol and ergocalciferol, vitamin E compounds include tocopherol, tocopherol acetate, tocopherol succinate, tocopherol nicotinate and tocotrienol, and vitamin K compounds include phytonadione and menatetrenone. The fat-soluble substance 1 may be used as a single type or a combination of two or more.

The mixing proportion of the fat-soluble substance 1 is preferably not greater than 60 parts by mass and more preferably not greater than 50 parts by mass with respect to 100 parts by mass of the sodium alginate aqueous solution, from the viewpoint of microdispersion of the fat-soluble substance 1. From the viewpoint of improving the yield of the microcapsules 100, the lower limit for the mixing proportion of the fat-soluble substance 1 is about 5 parts by mass.

The concentration of the sodium alginate aqueous solution 2 is preferably 0.1-5.0 mass%, more preferably 0.5-3.0 mass% and even more preferably 0.5-2.0 mass%. If the concentration of the sodium alginate aqueous solution 2 is less than 0.1 mass%, gelling during the spraying step described hereunder will tend to be more difficult, and if it exceeds 5.0 mass%, the emulsified liquid 5 will not readily flow in the supply passage during the spraying step, and it will not easily be sprayed by the nozzle.

The viscosity of the sodium alginate aqueous solution 2 at 25°C is preferably at least 5 mPa·s, more preferably 5-2000 mPa_{·}s, even more preferably 10-500 mPa·s and yet more preferably 15-100 mPa·s. If the viscosity of the sodium alginate aqueous solution 2 is less than 5 mPa·s, the durability of the microcapsules will tend to be lowered.

A more stable emulsified liquid 5 can be formed by adding an emulsifier as necessary, when the fat-soluble substance 1 and sodium alginate aqueous solution 2 are mixed. The emulsifier is not particularly restricted so long as it is one used for medicines, foods and drinks, and examples include glycerin fatty acid esters, glycerin acetate fatty acid esters, glycerin lactate fatty acid esters, glycerin succinate fatty acid esters, glycerin diacetyltartrate fatty acid esters, sorbitan fatty acid esters, sucrose fatty acid esters, sucrose acetate isobutyric acid ester, polyglycerin fatty acid esters, polyglycerin-condensed ricinoleic acid ester, propyleneglycol fatty acid esters, calcium stearoyl lactate, sodium stearoyl lactate, polyoxyethylenesorbitan monostearate, polyoxyethylenesorbitan monoglyceride and lecithin. The amount of emulsifier added is about 0.1-5 parts by mass with respect to 100 parts by mass of the sodium alginate aqueous solution.

### <Spraying step>

Next, the emulsified liquid 5 is sprayed as a mist into the calcium ion-containing solution 15 through a nozzle 7, as shown in Fig. 1(c), to form microcapsules 100 having the emulsified particles 10 composed of the fat-soluble substance 1 encapsulated in the calcium alginate gel 20. That is, the droplets of the emulsified liquid 5 contact with the calcium ion-containing solution 15, to obtain microcapsules 100.

In other words, the calcium ion-containing solution 15 functions as a gelling agent (coagulant), and when the emulsified liquid 5 is sprayed in the calcium ion-containing solution 15, the sodium alginate on the surface of the sprayed emulsified liquid 5 reacts with the calcium ion, forming a gel of insoluble calcium alginate. As a result, the emulsified particles 10 are encapsulated in the calcium alginate gel 20, forming microcapsules 100.

The calcium ion-containing solution 15 is preferably a calcium chloride aqueous solution, calcium lactate aqueous solution or calcium sulfate aqueous solution, from the viewpoint of instantaneous gelling, and it is preferably a calcium chloride aqueous solution from the viewpoint of easier release of calcium ion.

The calcium ion concentration of the calcium ion-containing solution 15 is preferably 0.5-20 mass% and more preferably 1-10 mass%. If the calcium ion concentration is less than 0.5 mass% it will tend to be more difficult to obtain a gel, and if it exceeds 20 mass% the cost will be increased and a longer time will tend to be necessary for the washing step described below.

The discharge slit diameter of the nozzle 7 is preferably not greater than 1.7 mm, more preferably not greater than 1.2 mm and even more preferably not greater than 1.1 mm. If the discharge slit diameter is greater than 1.7 mm, it may not be possible to obtain reduced particle sizes for the microcapsules 100. The nozzle 7 may have a single discharge slit, or more than one.

The spraying gas pressure through the nozzle 7 during spraying of the emulsified liquid 5 is preferably 0.1-1.0 MPa, more preferably 0.1-0.5 MPa and even more preferably 0.3-0.5 MPa. If the pressure is less than 0.1 MPa the particle sizes of the microcapsules 100 will tend to increase, and if it is greater than 1.0 MPa, concavities and convexities will be produced in the microcapsules 100, and the degree of deformation will tend to be increased. The liquid conveyance speed of the emulsified liquid 5 through the nozzle 7 is preferably 0.1-2.0 mL/min and more preferably 0.25-1.0 mL/min. If the liquid conveyance speed is less than 0.1 mL/min the production efficiency will tend to be lower, and if it is greater than 2.0 mL/min the particle sizes of the microcapsules 100 will tend to be larger.

### <Washing step>

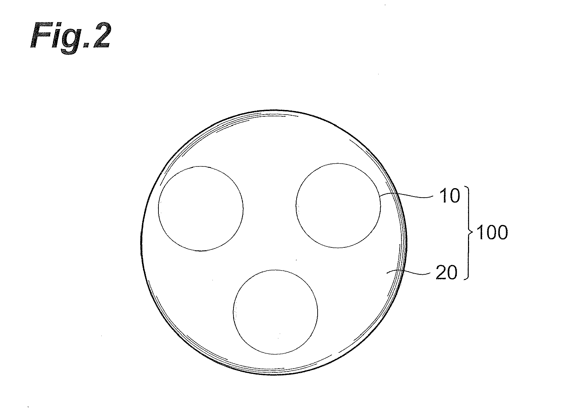

Next, the microcapsules 100 are filtered and collected, and appropriate washing treatment and classification are carried out depending on the purpose of use, for isolation as O/W bilayer microcapsules. Fig. 2 is a schematic view showing microcapsules 100 formed by the production method according to an embodiment of the invention. According to this embodiment, therefore, the emulsified particles 10 composed of the fat-soluble substance 1 as the core substance are homogeneously encapsulated in the calcium alginate gel 20 and spherical microcapsules 100 with small particle sizes and a low degree of deformation can be obtained.

The mean particle size of the microcapsules 100 is preferably less than 100 µm, more preferably not greater than 50 µm and more preferably not greater than 30 µm. If the mean particle size of the microcapsules 100 is 100 µm or greater, and they are added to foods or drinks, the foods or drinks will tend to have an inferior feel of the microcapsules in the mouth/throat and stomach. The mean particle size of the microcapsules 100 can be measured using a laser diffraction/scattering particle size distribution meter, and it is referred to as the volume-average particle size.

The degree of deformation of the microcapsules 100 is preferably less than 1.20, more preferably less than 1.15 and even more preferably less than 1.10. With the degree of deformation of 1.20 or greater, the durability of the microcapsules 100 will tend to be lower. The degree of deformation is the value determined by measuring the long diameter (the longest diameter of the microcapsules) and the short diameter (the shortest diameter of the microcapsules) from a photograph of the microcapsules 100 taken with an optical microscope, and dividing the long diameter by the short diameter. That is, the degree of deformation approaching 1.00 is more spherical.

The microcapsules 100 preferably have a narrow particle size distribution, and preferably the content ratio of microcapsules with particle sizes of 100 µm or greater can be adequately reduced during production. A narrower particle size distribution of the microcapsules 100 will allow more efficient collection of microcapsules with particle sizes of less than 100 µm, so that foods and drinks containing the microcapsules can have improved feel of the microcapsules in the mouth/throat and stomach.

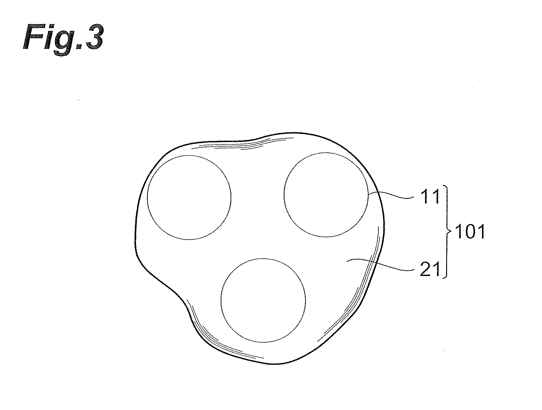

Fig. 3 is a schematic view showing microcapsules 101 formed by a conventional method, wherein the emulsified particles 11 are encapsulated in the calcium alginate gel 21, but not homogeneously. With microcapsules obtained in the comparative examples described hereunder, the degree of deformation is high as shown in Fig. 3, and the encapsulated emulsified particles 11 reside more easily near the surface of the calcium alginate gel 21, so that the durability tends to be lower.

The contained ratio of the fat-soluble substance encapsulated in the microcapsules is preferably at least 55%. The contained ratio is calculated as the content of the fat-soluble substance in the dry microcapsules, obtained by drying the microcapsules under prescribed drying conditions, adding ethanol, crushing, centrifuging the ethanol solution containing the crushed microcapsules, and then measuring the absorbance.

The microcapsules of this embodiment can be used in medicines, functional foods and drinks, or food and drink additives, by appropriately varying the encapsulated fat-soluble substance. They are particularly suitable for addition to foods and drinks because they have small particle sizes and are spherical. The foods and drinks containing the microcapsules therefore have sufficiently excellent feel of the microcapsules in the mouth/throat and stomach. Moreover, since the microcapsules also have excellent durability, leakage of fat-soluble substances into the foods and drinks can be inhibited, thus preventing reduction in the quality of the foods and drinks.

The present invention is not in any way limited to the preferred embodiment described above.

### Examples

The present invention will now be explained in detail by examples, with the understanding that the invention is not limited to the examples.

### (Example 1)

After mixing 85.5 g of a 1 mass% sodium alginate aqueous solution comprising sodium alginate (product of Wako Pure Chemical Industries, Ltd.) dissolved in distilled water, 4.5 g of an emulsifier (trade name: "tween80", product of Wako Pure Chemical Industries, Ltd.) and 10 g of vitamin E (product of Wako Pure Chemical Industries, Ltd.), the mixture was pre-emulsified at 60°C using a homomixer (trade name: "BM-2", product of Nippon Seiki Co., Ltd.) at 8000 rpm for 10 minutes. The pre-emulsified liquid was then emulsified at 20 MPa, 60°C using a continuous high-pressure homogenizer (trade name: "Homogenizer OA-06-075S", product of Izumi Food Machinery Co., Ltd.), to obtain an O/W emulsified liquid with a weight-average particle size of 170 nm.

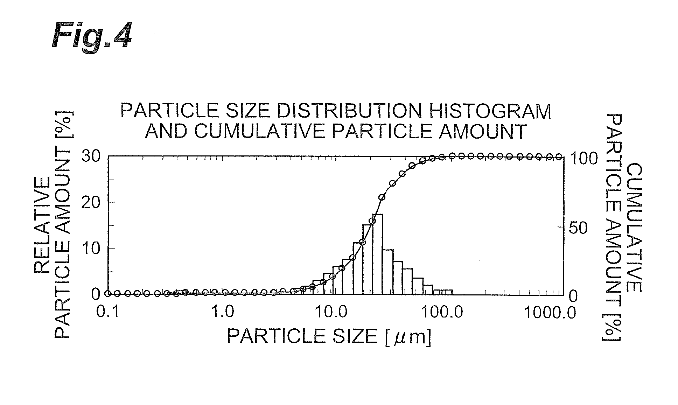

Next, the O/W emulsified liquid was sprayed through a spray nozzle (trade name: "Model AM-6", product of Atmax, Inc., discharge slit diameter: 1.1 mm) into a 5 mass% calcium chloride aqueous solution at a liquid conveyance speed of 1.0 mL/min and a spraying gas pressure of 0.3 MPa, to form O/W bilayer microcapsules. The O/W bilayer microcapsules were filtered with 5A filter paper (product of Advantech Toyo Kaisha, Ltd.) for collection. The collected O/W bilayer microcapsules were rinsed with a 3-fold amount of distilled water and then refiltered with 5A filter paper for collection. The volume-average particle size of the obtained O/W bilayer microcapsules was 25 µm, and the results of measuring the particle size distribution are shown in Fig. 4.

### (Example 2)

The same procedure was carried out as in Example 1, except for using a 0.5 mass% sodium alginate aqueous solution, to obtain an O/W emulsified liquid with a weight-average particle size of 264 nm. This O/W emulsified liquid was used for the same procedure as in Example 1, and the O/W bilayer microcapsules were collected. The volume-average particle size of the obtained O/W bilayer microcapsules was 25 µm.

### (Example 3)

The same procedure was carried out as in Example 1, except that the liquid conveyance speed was 0.25 mL/min and the spraying gas pressure was 0.5 MPa, during spraying of the O/W emulsified liquid, and the O/W bilayer microcapsules were collected. The volume-average particle size of the obtained O/W bilayer microcapsules was 21 µm.

### (Comparative Example 1)

After mixing 85.5 g of a 1 mass% sodium alginate aqueous solution comprising sodium alginate (product of Wako Pure Chemical Industries, Ltd.) dissolved in distilled water, 4.5 g of an emulsifier (trade name: "tween80", product of Wako Pure Chemical Industries, Ltd.) and 10 g of vitamin E (product of Wako Pure Chemical Industries, Ltd.), the mixture was emulsified under ice-cold conditions using a homomixer (trade name: "BM-2", product of Nippon Seiki Co., Ltd.) at 8000 rpm for 10 minutes, to obtain an O/W emulsified liquid with a weight-average particle size of 849 nm.

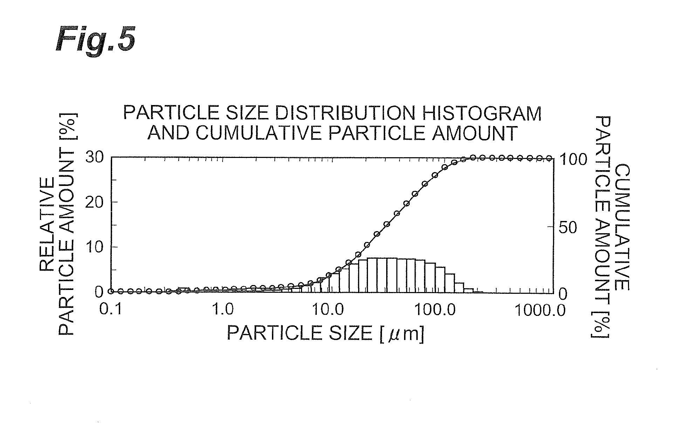

The O/W emulsified liquid was sprayed through a spray nozzle (trade name: "Model AM-6", product of Atmax, Inc., discharge slit diameter: 1.1 mm) into a 5 mass% calcium chloride aqueous solution under conditions with a liquid conveyance speed of 1.0 mL/min and a spraying gas pressure of 0.3 MPa, to form O/W bilayer microcapsules. The O/W bilayer microcapsules were filtered with 5A filter paper (product of Advantech Toyo Kaisha, Ltd.) for collection. The collected O/W bilayer microcapsules were rinsed with a 3-fold amount of distilled water and then refiltered with 5A filter paper for collection. The volume-average particle size of the obtained O/W bilayer microcapsules was 31 µm, and the results of measuring the particle size distribution are shown in Fig. 5.

### (Example 4)

The same procedure was carried out as in Example 1, except for changing the emulsification pressure to 5 MPa, to obtain an O/W emulsified liquid with a weight-average particle size of 359 nm. This O/W emulsified liquid was used for the same procedure as in Example 1, and the O/W bilayer microcapsules were collected. The volume-average particle size of the obtained O/W bilayer microcapsules was 95 µm.

### (Example 5)

The same procedure was carried out as in Example 1, except for using a nanomizer (trade name: "NM2-L200") by Yoshida Kikai Co. Ltd. and changing the emulsification pressure to 40 MPa, to obtain an O/W emulsified liquid with a weight-average particle size of 378 nm. This O/W emulsified liquid was used for the same procedure as in Example 1, and the O/W bilayer microcapsules were collected. The volume-average particle size of the obtained O/W bilayer microcapsules was 38 µm.

### (Example 6)

The same procedure was carried out as in Example 5, except for changing the emulsification pressure to 100 MPa, to obtain an O/W emulsified liquid with a weight-average particle size of 339 nm. This O/W emulsified liquid was used for the same procedure as in Example 1, and the O/W bilayer microcapsules were collected. The volume-average particle size of the obtained O/W bilayer microcapsules was 39 µm.

### (Example 7)

The same procedure was carried out as in Example 5, except for changing the emulsification pressure to 200 MPa, to obtain an O/W emulsified liquid with a weight-average particle size of 279 nm. This O/W emulsified liquid was used for the same procedure as in Example 1, and the O/W bilayer microcapsules were collected. The volume-average particle size of the obtained O/W bilayer microcapsules was 40 µm.

### (Example 8)

The same procedure was carried out as in Example 1, except for changing the concentration of the sodium alginate aqueous solution to 0.5 mass% and using a sodium alginate aqueous solution with a different viscosity than Example 1, to obtain an O/W emulsified liquid with a weight-average particle size of 417 nm. This O/W emulsified liquid was used for the same procedure as in Example 1, and the O/W bilayer microcapsules were collected. The volume-average particle size of the obtained O/W bilayer microcapsules was 44 µm.

### (Example 9)

The same procedure was carried out as in Example 1, except for changing the concentration of the sodium alginate aqueous solution to 1.60 mass% and using a sodium alginate aqueous solution with a different viscosity than Example 1, to obtain an O/W emulsified liquid with a weight-average particle size of 745 nm. This O/W emulsified liquid was used for the same procedure as in Example 1, and the O/W bilayer microcapsules were collected. The volume-average particle size of the obtained O/W bilayer microcapsules was 27 µm.

### (Example 10)

The same procedure was carried out as in Example 1, except for changing the concentration of the sodium alginate aqueous solution to 2.00 mass% and using a sodium alginate aqueous solution with a different viscosity than Example 1, to obtain an O/W emulsified liquid with a weight-average particle size of 419 nm. This O/W emulsified liquid was used for the same procedure as in Example 1, and the O/W bilayer microcapsules were collected. The volume-average particle size of the obtained O/W bilayer microcapsules was 39 µm.

### (Example 11)

The same procedure was carried out as in Example 1, except that the spraying was into a 2.5 mass% calcium lactate aqueous solution during spraying of the O/W emulsified liquid, and the O/W bilayer microcapsules were collected. The volume-average particle size of the obtained O/W bilayer microcapsules was 56 µm.

### (Example 12)

The same procedure was carried out as in Example 1, except that the spraying was into a 2.5 mass% calcium sulfate aqueous solution during spraying of the O/W emulsified liquid, and the O/W bilayer microcapsules were collected. The volume-average particle size of the obtained O/W bilayer microcapsules was 77 µm.

### (Example 13)

The same procedure was carried out as in Example 1, except that the spraying gas pressure was 0.1 MPa, during spraying of the O/W emulsified liquid, and the O/W bilayer microcapsules were collected. The volume-average particle size of the obtained O/W bilayer microcapsules was 94 µm.

### (Example 14)

The same procedure was carried out as in Example 1, except that the spraying gas pressure was 0.5 MPa, during spraying of the O/W emulsified liquid, and the O/W bilayer microcapsules were collected. The volume-average particle size of the obtained O/W bilayer microcapsules was 38 µm.

### (Example 15)

The same procedure was carried out as in Example 1, except that the nozzle discharge slit diameter was 1.2 mm, during spraying of the O/W emulsified liquid, and the O/W bilayer microcapsules were collected. The volume-average particle size of the obtained O/W bilayer microcapsules was 98 µm.

### (Example 16)

The same procedure was carried out as in Example 1, except that the liquid conveyance speed was 0.3 mL/min, the spraying gas pressure was 0.5 MPa and the nozzle discharge slit diameter was 1.7 mm, during spraying of the O/W emulsified liquid, and the O/W bilayer microcapsules were collected. The volume-average particle size of the obtained O/W bilayer microcapsules was 66 µm.

### (Example 17)

The same procedure was carried out as in Example 1, except that the calcium ion concentration of the calcium ion-containing solution was changed to 0.5 mass% during spraying of the O/W emulsified liquid, and the O/W bilayer microcapsules were collected. The volume-average particle size of the obtained O/W bilayer microcapsules was 69 µm.

### (Example 18)

The same procedure was carried out as in Example 1, except that the calcium ion concentration of the calcium ion-containing solution was changed to 10 mass% during spraying of the O/W emulsified liquid, and the O/W bilayer microcapsules were collected. The volume-average particle size of the obtained O/W bilayer microcapsules was 50 µm.

### (Example 19)

The same procedure was carried out as in Example 1, except that the calcium ion concentration of the calcium ion-containing solution was changed to 20 mass% during spraying of the O/W emulsified liquid, and the O/W bilayer microcapsules were collected. The volume-average particle size of the obtained O/W bilayer microcapsules was 50 µm.

### <Measurement of particle size and particle size distribution>

The particle size of the emulsified particles in the O/W emulsified liquid was measured using a dynamic light scattering distribution meter (trade name: "ELS-8000", product of Otsuka Electronics Co., Ltd.). The particle size and particle size distribution of the O/W bilayer microcapsules were measured using a laser diffraction/scattering particle size distribution meter (trade name: "SALD-3000", product of Shimadzu Corp.).

### <Microscope observation>

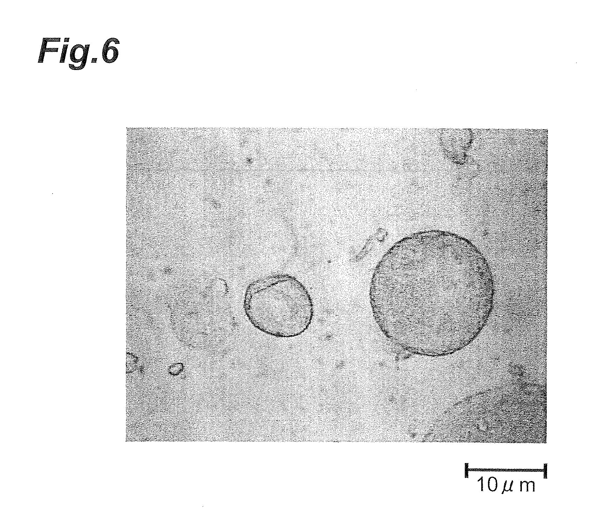

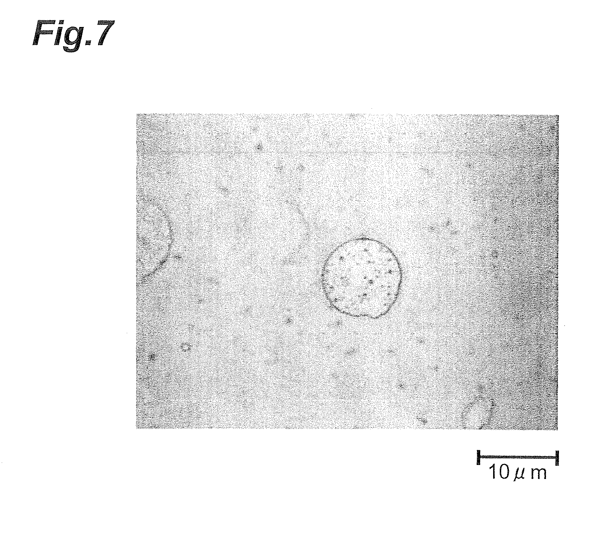

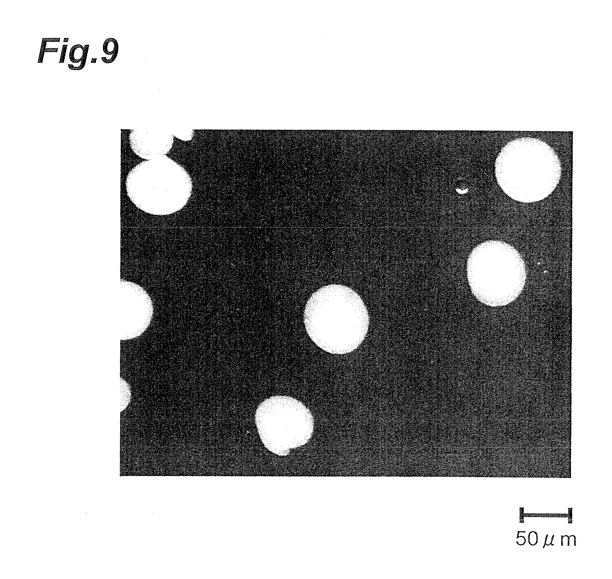

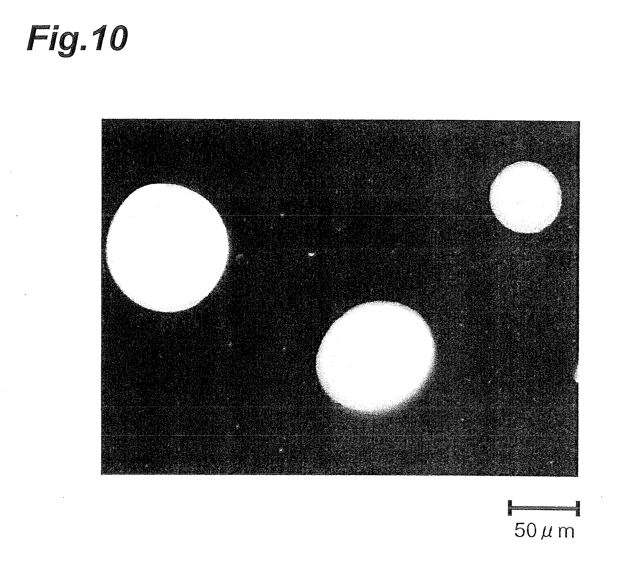

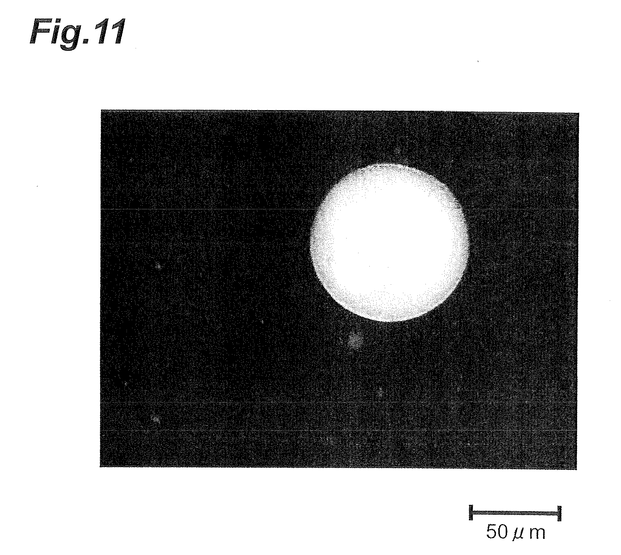

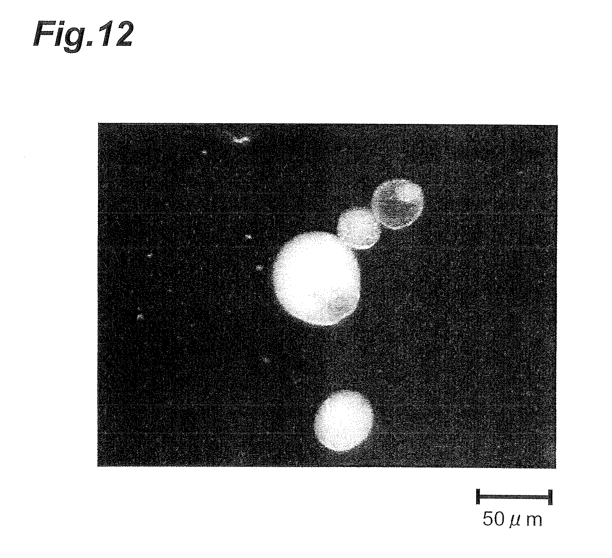

The O/W bilayer microcapsules obtained in Example 1, Example 2 and Comparative Example 1 were observed under an optical microscope (trade name: "BX-51-PRF", product of Olympus Corp.). Figs. 6 to 8 are optical microscope photographs of the O/W bilayer microcapsules obtained in Example 1, Example 2 and Comparative Example 1. The O/W bilayer microcapsules of Example 13, Example 15, Example 16 and Example 18 were observed with a digital microscope (trade name: "Digital Microscope VHX-100F", product of Keyence Corp.). Figs. 9 to 12 are photographs of the O/W bilayer microcapsules obtained in Example 13, Example 15, Example 16 and Example 18.

### <Degree of deformation of O/W bilayer microcapsules>

The long diameter and short diameter of the obtained O/W bilayer microcapsules were measured from the optical microscope photograph, and the degree of deformation was calculated. The degree of deformation was measured for 50 O/W bilayer microcapsules, and the average of the 50 degrees of deformation was recorded as the degree of deformation for the example.

### <Measurement of contained ratio of vitamin E in O/W bilayer microcapsules>

The O/W bilayer microcapsules obtained in Examples 1-19 and Comparative Example 1 were dried at 105°C for 6 hours, and the dry weight was measured, ethanol was added, and the mixture was stirred at 700 rpm for 12 hours and then treated for 40 minutes with an ultrasonic wave homogenizer (trade name: "VP-050", product of Teitech Co., Ltd.) for crushing. The ethanol solution containing crushed microcapsules was centrifuged at 7000 rpm for 10 minutes, the supernatant was taken, and the absorbance at 285 nm was measured using a spectrophotometer (trade name: "spectrophotometer U-3210", product of Hitachi Instruments Service Co., Ltd.). The vitamin E concentration was determined from the measured absorbance, and the contained ratio of the vitamin E in the dried O/W bilayer microcapsules was calculated.

### <Durability test for O/W bilayer microcapsules>

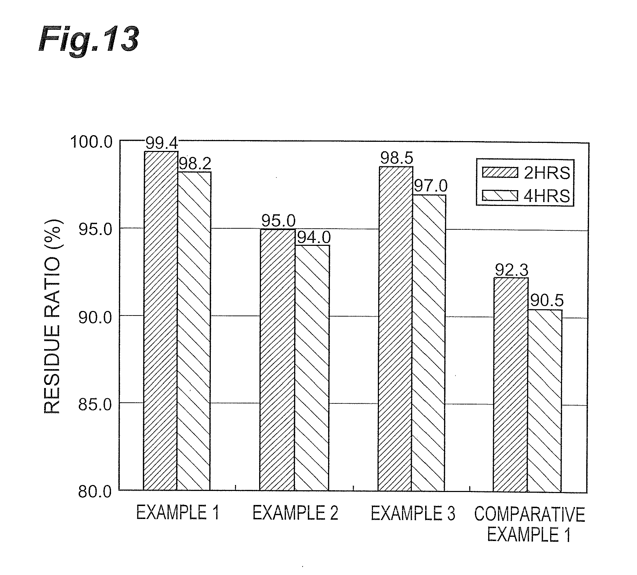

The O/W bilayer microcapsules obtained in Examples 1-3 and Comparative Example 1 were suspended in distilled water, and a durability test was conducted by shaking for a prescribed time with a shaker (trade name: "SA-31", product of Yamato Scientific Co., Ltd.) at a speed of 240 rpm. Next, using the same procedure as for measurement of the contained ratio of the vitamin E described above, the contained ratio of the vitamin E in the dried O/W bilayer microcapsules was calculated at 2 and 4 hours of shaking, and the vitamin E residue ratio was calculated with the contained ratio of the vitamin E before shaking as 100%. The vitamin E residue ratio was similarly calculated at 4 hours of shaking for Examples 4-19 as well. The results are shown in Fig. 13.

Tables 1-4 show the preparation conditions and the results of each measurement (mean particle size, degree of deformation, contained ratio of vitamin E and durability), for the O/W bilayer microcapsules obtained in Examples 1-19 and Comparative Example 1.

**[Table 1]**

| | | Example 1 | Example 2 | Example 3 | Comp. Ex. 1 |
|---|---|---|---|---|---|
| Pre-emulsification conditions | | 8000 rpm 10 min/60°C | 8000 rpm 10 min/60°C | 8000 rpm 10 min/60°C | 8000 rpm 10 min/ice-cold |
| Emulsification conditions | | 20MPa, 60°C | 20MPa, 60°C | 20MPa, 60°C | - |
| Sodium alginate concentration (mass%) | | 1.0 | 0.5 | 1.0 | 1.0 |
| Sodium alginate viscosity (mPa·s) | | 40 | 20 | 40 | 40 |
| Mean particle size of emulsified particles (nm) | | 170 | 264 | 250 | 849 |
| Spraying conditions | Liquid conveyance speed (ml/min) | 1.0 | 1.0 | 0.25 | 1.0 |
| | Spraying gas pressure (MPa) | 0.3 | 0.3 | 0.5 | 0.3 |
| | Nozzle discharge slit diameter (mm) | 1.1 | 1.1 | 1.1 | 1.1 |
| Ca ion-containing solution type | | Calcium chloride | Calcium chloride | Calcium chloride | Calcium chloride |
| Ca ion concentration (mass%) | | 5 | 5 | 5 | 5 |
| Mean particle size of microcapsules (µm) | | 25 | 25 | 21 | 31 |
| Degree of deformation (long diameter/short diameter) | | 1.07 | 1.13 | 1.12 | 1.20 |
| Contained ratio of vitamin E (%) | | 57.4 | 60.4 | 56.5 | 54.9 |
| Durability (after 2 hours) (%) | | 99.4 | 95.0 | 98.5 | 92.3 |
| Durability (after 4 hours) (%) | | 98.2 | 94.0 | 97.0 | 90.5 |

**[Table 2]**

| | | Example 4 | Example 5 | Example 6 | Example 7 | Example 8 | Example 9 | Example 10 |
|---|---|---|---|---|---|---|---|---|
| Pre-emulsification conditions | | 8000 rpm 10min/60°C | 8000 rpm 10 min/60°C | 8000 rpm 10 min/60°C | 8000 rpm 10min/60°C | 8000 rpm 10min/60°C | 8000 rpm 10min/60°C | 8000 rpm 10 min/60°C |
| Emulsification conditions | | 5 MPa, 60°C | 40 MPa, 60°C | 100 MPa, 60°C | 200 MPa, 60°C | 20 MPa, 60°C | 20 MPa, 60°C | 20 MPa, 60°C |
| Sodium alginate concentration (mass%) | | 1.0 | 1.0 | 1.0 | 1.0 | 0.5 | 1.60 | 2.00 |
| Sodium alginate viscosity (mPa·s) | | 40 | 40 | 40 | 40 | 5 | 1000 | 2000 |
| Mean particle size of emulsified particles (nm) | | 359 | 378 | 339 | 279 | 417 | 745 | 419 |
| Spraying conditions | Liquid conveyance speed (ml/min) | 1.0 | 1.0 | 1.0 | 1.0 | 1.0 | 1.0 | 1.0 |
| | Spraying gas pressure (MPa) | 0.3 | 0.3 | 0.3 | 0.3 | 0.3 | 0.3 | 0.3 |
| | Nozzle discharge slit diameter (mm) | 1.1 | 1.1 | 1.1 | 1.1 | 1.1 | 1.1 | 1.1 |
| Ca ion-containing solution type | | Calcium chloride | Calcium chloride | Calcium chloride | Calcium chloride | Calcium chloride | Calcium chloride | Calcium chloride |
| Ca ion concentration (mass%) | | 5 | 20 | 20 | 20 | 5 | 5 | 5 |
| Mean particle size of microcapsules (µm) | | 95 | 38 | 39 | 40 | 44 | 27 | 39 |
| Degree of deformation (long diameter/short diameter) | | 1.13 | 1.16 | 1.18 | 1.12 | 1.13 | 1.06 | 1.11 |
| Contained ratio of vitamin E (%) | | 54.1 | 75.1 | 76.9 | 77.5 | 34.1 | 54.0 | 56.7 |
| Durability (after 4 hours) (%) | | 93.6 | 94.6 | 97.0 | 97.3 | 91.6 | 98.7 | 99.0 |

**[Table 3]**

| | | Example 11 | Example 12 | Example 13 | Example 14 |
|---|---|---|---|---|---|
| Pre-emulsification conditions | | 8000 rpm 10 min/60°C | 8000 rpm 10 min/60°C | 8000 rpm 10 min/60°C | 8000 rpm 10 min/60°C |
| Emulsification conditions | | 20MPa, 60°C | 20MPa, 60°C | 20MPa, 60°C | 20MPa, 60°C |
| Sodium alginate concentration (mass%) | | 1.0 | 1.0 | 1.0 | 1.0 |
| Sodium alginate viscosity (mPa·s) | | 40 | 40 | 40 | 40 |
| Mean particle size of emulsified particles (nm) | | 170 | 170 | 170 | 170 |
| Spraying conditions | Liquid conveyance speed (ml/min) | 1.0 | 1.0 | 1.0 | 1.0 |
| | Spraying gas pressure (MPa) | 0.3 | 0.3 | 0.1 | 0.5 |
| | Nozzle discharge slit diameter (mm) | 1.1 | 1.1 | 1.1 | 1.1 |
| Ca ion-containing solution type | | Calcium lactate | Calcium sulfate | Calcium chloride | Calcium chloride |
| Ca ion concentration (mass%) | | 2.5 | 2.5 | 5 | 5 |
| Mean particle size of microcapsules (µm) | | 56 | 77 | 94 | 38 |
| Degree of deformation (long diameter/short diameter) | | 1.08 | 1.10 | 1.15 | 1.16 |
| Contained ratio of vitamin E (%) | | 53.5 | 60.9 | 53.0 | 60.7 |
| Durability (after 4 hours) (%) | | 94.5 | 95.2 | 97.3 | 96.0 |

**[Table 4]**

| | | Example 15 | Example 16 | Example 17 | Example 18 | Example 19 |
|---|---|---|---|---|---|---|
| Pre-emulsification conditions | | 8000 rpm 10min/60°C | 8000 rpm 10min/60°C | 8000 rpm 10 min/60°C | 8000 rpm 10 min/60°C | 8000 rpm 10min/60°C |
| Emulsification conditions | | 20MPa, 60°C | 20MPa, 60°C | 20MPa, 60°C | 20MPa, 60°C | 20MPa, 60°C |
| Sodium alginate concentration (mass%) | | 1.0 | 1.0 | 1.0 | 1.0 | 1.0 |
| Sodium alginate viscosity (mPa·s) | | 40 | 40 | 40 | 40 | 40 |
| Mean particle size of emulsified particles (nm) | | 170 | 170 | 170 | 170 | 170 |
| Spraying conditions | Liquid conveyance speed (ml/min) | 1.0 | 0.3 | 1.0 | 1.0 | 1.0 |
| | Spraying gas pressure (MPa) | 0.3 | 0.5 | 0.3 | 0.3 | 0.3 |
| | Nozzle discharge slit diameter (mm) | 1.2 | 1.7 | 1.1 | 1.1 | 1.1 |
| Ca ion-containing solution type | | Calcium chloride | Calcium chloride | Calcium chloride | Calcium chloride | Calcium chloride |
| Ca ion concentration (mass%) | | 5 | 5 | 0.5 | 10 | 20 |
| Mean particle size of microcapsules (µm) | | 98 | 66 | 69 | 50 | 50 |
| Degree of deformation (long diameter/short diameter) | | 1.16 | 1.15 | 1.19 | 1.19 | 1.17 |
| Contained ratio of vitamin E (%) | | 64.2 | 71.6 | 62.6 | 62.2 | 49.7 |
| Durability (after 4 hours) (%) | | 93.8 | 95.9 | 99.5 | 94.4 | 94.6 |

The results showed that the O/W bilayer microcapsules obtained in Examples 1-19 were spherical, with small mean particle sizes and low degree of deformation. Figs. 4 and 5 confirmed that the O/W bilayer microcapsules obtained in Example 1 had a narrower particle size distribution than the O/W bilayer microcapsules obtained in Comparative Example 1. Also, Fig. 13 and Tables 1-4 confirmed that the O/W bilayer microcapsules obtained in Examples 1-19 had sufficiently excellent durability as well.

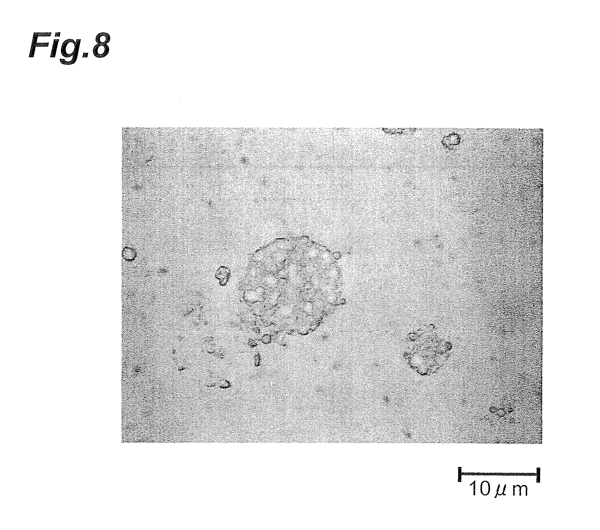

On the other hand, the O/W bilayer microcapsules obtained in Comparative Example 1 had a larger mean particle size than the O/W bilayer microcapsules obtained in Examples 1-19, with easy formation of concavities and convexities during spraying and a wide particle size distribution. In addition, the O/W bilayer microcapsules obtained in Comparative Example 1 were not spherical, as shown in Fig. 8, while they had vitamin E present near the surface of the calcium alginate gel, and also inferior durability.

### Reference Signs List

1: Fat-soluble substance, 2: sodium alginate aqueous solution, 5: emulsified liquid, 7: nozzle, 10, 11: emulsified particles, 15: calcium ion-containing solution, 20, 21: calcium alginate gels, 100, 101: microcapsules.

## Claims

1. A method of producing microcapsules, comprising:
an emulsification step of mixing a fat-soluble substance and a sodium alginate aqueous solution to obtain an emulsified liquid dispersing emulsified particles composed of the fat-soluble substance and having a mean particle size of not greater than 800 nm; and
a spraying step of spraying the emulsified liquid into a calcium ion-containing solution to obtain microcapsules encapsulating the emulsified particles.

2. A method of producing microcapsules, comprising a step of contacting droplets of an emulsified liquid comprising emulsified particles composed of a fat-soluble substance and having a mean particle size of not greater than 800 nm dispersed in a sodium alginate aqueous solution, with a calcium ion-containing solution, to obtain microcapsules having emulsified particles encapsulated in a calcium alginate gel.

3. The method of producing microcapsules according to claim 1 or 2, wherein the emulsified particles are dispersed under pressure.

4. The method of producing microcapsules according to claim 3, wherein the emulsified particles are dispersed under pressure of 5 MPa or greater.

5. The method of producing microcapsules according to any one of claims 1 to 4, wherein a viscosity of the sodium alginate aqueous solution is 5 mPa·s or greater.

6. The method of producing microcapsules according to any one of claims 1 to 5, wherein the calcium ion-containing solution is a calcium chloride aqueous solution, a calcium lactate aqueous solution or a calcium sulfate aqueous solution.

7. Microcapsules obtainable by the method of producing microcapsules according to any one of claims 1 to 6.

8. Microcapsules encapsulating a fat-soluble substance,
the microcapsules having a mean particle size of less than 100 µm and a degree of deformation of less than 1.20.

9. A food or drink containing the microcapsules according to claim 7 or 8.
